# EUROPÄISCHE PATENTSCHRIFT

(11) **EP 1 343 743 B1**
(45) Veröffentlichungstag und Bekanntmachung des Hinweises auf die Patenterteilung: **22.09.2004**
(21) Anmeldenummer: 01985854.7
(22) Anmeldetag: 07.12.2001
(51) Int. Cl.: C07C 27/06

(54) **VERFAHREN ZUR HERSTELLUNG VON TETRAHYDROFURAN**
METHOD FOR THE PRODUCTION OF TETRAHYDROFURAN
PROCEDE DE PRODUCTION DE TETRAHYDROFURANE

(30) Priorität: 11.12.2000 DE 10061556
(43) Veröffentlichungstag der Anmeldung: 17.09.2003
(73) Patentinhaber: BASF AKTIENGESELLSCHAFT, 67056 Ludwigshafen (DE)
(72) Erfinder: FISCHER, Rolf-Hartmuth, 69121 Heidelberg (DE); STEIN, Frank, 67098 Bad Dürkheim (DE); PINKOS, Rolf, 67089 Bad Dürkheim (DE); HESSE, Michael, 67549 Worms (DE); SPRAGUE, Michael, Jolyon, 68161 Mannheim (DE); RÖSCH, Markus, 55276 Oppenheim (DE); BORCHERT, Holger, 67591 Offstein (DE); SCHLITTER, Stephan, 67117 Limburgerhof (DE); RAHN, Ralf-Thomas, 68167 Mannheim (DE); WECK, Alexander, 67251 Freinsheim (DE)
(74) Vertreter: Isenbruck, Günter, Dr.
(86) Internationale Anmeldenummer: PCT/EP2001/014394
(87) Internationale Veröffentlichungsnummer: WO 2002/048128

(56) Entgegenhaltungen:
- EP-A- 0 322 140
- EP-A- 0 589 314
- PATENT ABSTRACTS OF JAPAN vol. 014, no. 548 (C-0785), 5. Dezember 1990 (1990-12-05) -& JP 02 233631 A (TONEN CORP), 17. September 1990 (1990-09-17) in der Anmeldung erwähnt
- PATENT ABSTRACTS OF JAPAN vol. 014, no. 548 (C-0785), 5. Dezember 1990 (1990-12-05) -& JP 02 233627 A (TONEN CORP), 17. September 1990 (1990-09-17) in der Anmeldung erwähnt

## Beschreibung

Die vorliegende Erfindung betrifft ein Verfahren zur Herstellung von gegebenenfalls alkylsubstituiertem Tetrahydrofuran durch katalytische Hydrierung in der Gasphase von Substraten, die ausgewählt sind aus der Gruppe bestehend aus Derivaten der Maleinsäure und Bemsteinsäure sowie diesen Säuren selbst. Unter Derivaten werden im Rahmen der vorliegenden Erfindung Anhydride verstanden, die ebenso wie die Säuren einen oder mehrere Alkylsubstituenten aufweisen können.

Die Herstellung von Tetrahydrofuran (THF) durch Gasphasenhydrierung von Maleinsäureanhydrid (MSA) ist eine seit vielen Jahren bekannte Reaktion. Zur Durchführung dieser katalytischen Reaktion sind in der Literatur zahlreiche Katalysatorsysteme beschrieben. Je nach Zusammensetzung des Katalysators und den gewählten Reaktionsparametern werden mit derartigen Katalysatoren unterschiedliche Produktverteilungen erreicht.

Es konnte gezeigt werden, daß bei der Hydrierung von MSA zu THF zunächst Bernsteinsäureanhydrid (BSA) und anschließend γ-Butyrolacton (GBL) als Zwischenstufen entstehen, wobei dies durch weitere Hydrierung zu 1,4-Butandiol (BDO) umgesetzt werden kann. Alle vorstehend genannten Zwischenprodukte können selbst als Edukte für die THF-Herstellung eingesetzt werden.

Sollen GBL und THF hergestellt werden, die Alkylsubstituenten aufweisen, so bietet es sich an, von den vorstehend genannten Edukten auch die entsprechenden alkylsubstituierten Spezies zu verwenden.

Die bei der Hydrierung verwendeten Katalysatoren sind, insbesondere bei älteren Verfahren, häufig chromhaltig. Dies findet seinen Niederschlag in der Patentliteratur, in der sich eine große Anzahl von Patenten und Patentanmeldungen finden, die chromhaltige Katalysatoren für die vorstehend beschriebene Hydrierreaktion offenbaren, wobei die Hydrierung in den meisten Fällen auf MSA als Edukt beschränkt ist. In der US 3,065,243 ist ein Verfahren offenbart, bei dem Kupferchromit als Katalysator dient. Laut Beschreibung und Beispielen entstehen bei dieser Reaktionsführung noch beträchtliche Mengen an BSA, das im Kreis gefahren werden muß. Wie bekannt ist, treten dabei häufig verfahrenstechnische Probleme aufgrund der Kristallisation des BSA oder auch daraus entstehender Bernsteinsäure mit anschließender Verstopfung von Rohrleitungen auf.

Die Offenbarung von weiteren Kupferchromit-Katalysatoren zur Hydrierung von MSA findet sich zum Beispiel in den Druckschriften US 3,580,930, US 4,006,165, der EP-A 638 565 sowie der WO 99/38856.

Die nach der US 5,072,009 verwendeten Katalysatoren entsprechen der allgemeinen Formel CuₗZn_{b}Al_{c}M_{d}Oₓ, in der M mindestens ein Element ist, das ausgewählt ist aus der Gruppe bestehend aus den Gruppen IIA und IIIA, VA, VIII, Ag, Au, den Gruppen IIIB bis VIIB sowie Lanthaniden und Aktiniden des Periodensystems der Elemente; b ist eine Zahl zwischen 0,001 und 500, c eine Zahl zwischen 0,001 und 500 und d eine Zahl von 0 bis <200 und x entspricht der Anzahl an Sauerstoffatomen, die nach den Valenzkriterien notwendig sind. Bei der Hydrierung von MSA mit den erfindungsgemäßen Katalysatoren entsteht nach den Beispielen, in denen ausschließlich chromhaltige Katalysatoren verwendet werden, THF in Ausbeuten von über 90%.

Die EP-A 0 404 408 offenbart einen Katalysator für die MSA-Hydrierung, dessen katalytisch aktives Material im wesentlichen dem Material, das in der vorstehend zitierten US 5,072,009 offenbart ist, entspricht. Es wird auf einem Träger. fixiert als Schalenkatalysator und nicht als Vollkatalysator eingesetzt. Im Gegensatz zu dem als Vollkatalysator vorliegenden Material entsteht hier hauptsächlich GBL nach den angegebenen Beispielen, in denen ebenfalls nur chromhaltige Katalysatoren zum Einsatz kommen.

Ein zweistufiges Katalysatorsystem zur Hydrierung von MSA ist in der Patentschrift US 5,149,836 beschrieben. Der Katalysator für die erste Stufe ist chromfrei, der Katalysator für die zweite Stufe basiert auf Cu-Zn-Cr-Oxiden.

Neuere Technologien rücken aufgrund der Toxizität mehr und mehr von der Verwendung chromhaltiger Katalysatoren ab. Beispiele für chromfreie Katalysatorsysteme finden sich in den Druckschriften WO 99/35139 (Cu-Zn-Oxid), WO 95/22539 (Cu-Zn-Zr) sowie der US 5,122,495 (Cu-Zn-Al-Oxid).

Ein ausschließlich aus Cu- und Al-Oxiden aufgebauter Katalysator für die MSA-Gasphasenhydrierung zu GBL wird in der WO 97/24346 offenbart. Dieser Katalysator enthält 50 bis 95 Gew.-%, vorzugsweise 80 bis 90 Gew.-% Kupferoxid, 3 bis 30 Gew.-%, vorzugsweise 5 bis 15 Gew.-% Aluminiumoxid sowie optional einen Binder. Bei der Hydrierung von MSA mit einem derartigen Katalysator werden Selektivitäten zu GBL bis zu ca. 98% erreicht.

Auch in der WO 91/16132 wird ein chromfreier Katalysator für die Hydrierung von MSA offenbart. Der Katalysator enthält 30 bis 65 Gew.-% CuO, 18 bis 50 Gew.-% ZnO und 8 bis 22 Gew.-% Al₂O₃. Vor dem Einsatz in die Hydrierreaktion wird der Katalysator unter einer Wasserstoffatmosphäre reduziert und anschließend unter einer Wasserstoffatmosphäre bei mindestens 400 °C und über wenigstens 8 Stunden aktiviert. Ein derartiger Katalysator liefert GBL-Selektivitäten von ca. 90%.

Castiglioni et al offenbaren in Catalysis Today 27 (1996), S. 181 bis 186 CuO/ZnO/Al₂O₃-Katalysatoren, die bei der Hydrierung von MSA hauptsächlich GBL liefern, es wird eine maximale THF-Selektivität von 17% beobachtet.

Die Verwendung eines Katalysators mit ähnlicher Zusammensetzung wie in der Patentanmeldung WO 97/24346 wird auch in der JP 2 233 631 offenbart. Das Ziel dieser Erfindung liegt darin, die Hydrierung von MSA so durchzuführen, daß als Hauptprodukte THF und BDO neben nur geringen oder gar keinen Mengen von GBL entstehen. Dieses wird dann durch die Verwendung der auf gemischten Cu-Al-Oxiden basierenden Katalysatoren sowie durch Einhalten bestimmter Reaktionsbedingungen erreicht. Generelle Mengenangaben bezüglich des Cu-und Al-Oxides finden sich nicht; in den Beispielen sind 2 Katalysatorzusammensetzungen offenbart, eine mit ca. 46 Gew.-% CuO und 33 Gew.-% Al₂O₃, die andere mit ca. 36 Gew.-% CuO und 47 Gew.-% Al₂O₃. Es wird bei Verwendung dieses Katalysators angeblich eine THF-Selektivität von bis zu 99% erreicht, wobei diese Selektivität nur dann erreicht wird, wenn mit einem Überschuß an GBL als Lösungsmittel gearbeitet wird. Wird die Hydrierung mit dem gleichen Katalysator in Abwesenheit des GBL durchgeführt, so sinkt die Selektivität auf 76%. Gemäß den Beispielen wird die Hydrierung bei Temperaturen zwischen ca. 210 bis 230 °C und GHSV-Werten von ca. 3200 bis 9600 durchgeführt. Die Wasserstoff/MSA-Verhältnisse liegen bei für industrielle Verfahren vergleichsweise ungünstig hohen Werten, in den Beispielen von 200 bis 800.

Die Hydrierung von MSA unter Bedingungen, die denen in der JP 2 233 631 entsprechen, jedoch unter Verwendung eines anderen Katalysators, werden in der JP 2 639 463 offenbart. Die Verwendung des Katalysators soll die Herstellung von BDO und THF durch Hydrierung von MSA ermöglichen. Eingesetzt wird hierbei ein Kupferoxid/Zinkoxid/Aluminiumoxid-Katalysator, dessen Zusammensetzung in der Beschreibung nicht quantitativ dargelegt wird. Die nach den Beispielen verwendeten Katalysatoren weisen eine Zusammensetzung von 20 Gew.-% CuO, 43,6Gew.-% ZnO und 18,1 Gew.-% Al₂O₃, 32,6 Gew.-% CuO 38,1 Gew.-% ZnO und 9,5 Gew.-% Al₂O₃, 24,2 Gew.-% CuO, 36,4 Gew.-% ZnO und 17,2 Gew.-% Al₂O₃, 26,4 Gew.-% CuO, 52,9 Gew.-%, ZnO, 7,6 Gew.-% Al₂O₃ und 1,4 Gew.-% CaO sowie 22,9 Gew.-% CuO, 44,8 Gew.-% ZnO und 16,3 Gew.-% Al₂O₃ auf. Es wird generell in einem Lösungsmittel wie GBL oder Dioxan gearbeitet, wobei eine maximale THF-Selektivität von 94% erreicht wird. Bei Durchführung der Reaktion ohne Lösungsmittel erreicht die THF-Selektivität einen maximalen Wert von 83%.

Die den oben zitierten Druckschriften zugrundeliegenden Technologien benutzen als Edukt für die Hydrierungsreaktionen vorgereinigtes MSA, das nach seiner Herstellung im allgemeinen durch Destillation von Verunreinigungen befreit wurde. MSA wird hergestellt durch partielle Oxidation von bestimmten Kohlenwasserstoffen, nämlich Benzol, Butengemischen sowie n-Butan, wobei dieses letztere vorzugsweise eingesetzt wird. Das Rohprodukt der Oxidation enthält neben dem gewünschten MSA vor allem Nebenprodukte wie Wasser, Kohlenmonoxid, Kohlendioxid, nicht umgesetzten Ausgangskohlenwasserstoff sowie Essig-und Acrylsäure, wobei diese Nebenprodukte unabhängig von den in die Oxidation eingesetzten Kohlenwasserstoffen sind. Zumeist werden die Nebenprodukte durch aufwendige Verfahren abgetrennt, beispielsweise durch Destillation, wie oben erwähnt. Das Reinigen erweist sich insbesondere als notwendig, weil die in den Hydrierverfahren eingesetzten Katalysatoren generell empfindlich auf solche Verunreinigungen reagieren. Die Desaktivierung der Katalysatoren ist bereits bei Einsatz von gereinigtem MSA ein Problem, da durch Belegung mit dessen Polymerisationsprodukten der Katalysator in der Regel in relativ kurzen Intervallen, die oftmals bei ca. 100 Stunden liegen, regeneriert werden muß. Die Tendenz zur Desaktivierung ist beim Vorliegen polymerisierbarer Verunreinigungen, wie beispielsweise der Acrylsäure, noch erhöht. Diese Tatsache ist dem Fachmann bekannt und wird beispielsweise auch in den Patentanmeldungen EP-A 322 140, WO 91/16132 und DE-OS 240 44 93 beschrieben.

Bis jetzt existiert im Stand der Technik lediglich eine Druckschrift, die die Hydrierung von lediglich grob vorgereinigtem MSA offenbart. Aus der WO 97/43234 ist bekannt, Maleinsäureanhydrid aus Maleinsäureanhydrid enthaltenden Gasströmen, die aus der Oxidation von Kohlenwasserstoffen stammen, mit Hilfe von mindestens 30 °C höher siedenden Absorptionsmitteln zu absorbieren, das Maleinsäureanhydrid aus diesen Absorptionsmitteln mit Hilfe von Wasserstoff herauszutreiben und den Maleinsäureanhydrid enthaltenden Wasserstoffstrom in der Gasphase an einem heterogenen Katalysator zu hydrieren. Dabei wird hauptsächlich BDO neben geringen Mengen GBL und THF erhalten. Die Hydrierung wird bei etwa 150 °C bis 300 °C und einem Druck von 5 bar bis 100 bar in der Gasphase durchgeführt. Als Katalysatoren werden promotierte Kupferkatalysatoren verwendet, wie sie in Journal of Catalysis 150, Seiten 177 bis 185 (1994) beschrieben sind. Dabei handelt es sich um chromhaltige Katalysatoren des Typs Cu/Mn/Ba/Cr und Cu/Zn/Mg/Cr. Somit werden gemäß der Offenbarung dieser Anmeldung chromhaltige Katalysatoren zur Hydrierung von MSA-Qualitäten, die die oben dargelegten Verunreinigungen aufweisen, eingesetzt. Der Einsatz chromhaltiger Katalysatoren wird jedoch aufgrund der Toxizität heutzutage so weit wie möglich vermieden. Zudem liefert das Verfahren neben dem Hauptprodukt BDO merkliche Mengen an nicht erwünschten Produkten, nämlich THF und GBL, die entweder abgetrennt und gereinigt oder in die Hydrierung zurückgeführt werden müssen. Dieser Aufwand ist häufig, insbesondere bei großtechnischen Verfahren, mit unerwünschten Kosten verbunden.

Welches der bei der Hydrierung von MSA zugänglichen Produkte GBL, THF und BDO das erwünschte Produkt ist, richtet sich oft nach dem Marktwachstum dieser Produkte oder deren Folgeprodukten oder auch der sonstigen Produktpalette des jeweiligen Herstellers. Es kann daher gesagt werden, daß manche Hersteller die Hydrierung von MSA zur Herstellung von THF benutzen, andere wiederum zur Gewinnung von GBL und/oder BDO.

Die Aufgabe der vorliegenden Erfindung liegt darin, ein Verfahren bereitzustellen, mit dem THF durch Hydrierung von MSA hergestellt werden kann. Dieses Verfahren soll dabei kontinuierlich und mit chromfreien Katalysatoren betrieben werden können und möglichst große Mengen des Wertprodukts THF liefern, um so eine größtmögliche Wirtschaftlichkeit zu erreichen. Weiterhin sollte der Katalysator mit MSA betrieben werden können, das nicht aufwendig vorgereinigt wurde, beispielsweise durch Destillation, und trotzdem eine hohe Standfestigkeit aufweisen, also kein häufiges Regenerieren erfordern.

Diese Aufgabe wird gelöst durch ein Verfahren zur Herstellung von gegebenenfalls alkylsubstituiertem THF durch katalytische Hydrierung in der Gasphase von C₄-Dicarbonsäuren und/oder deren Derivaten mit einem Katalysator enthaltend < 80 Gew.-%, vorzugsweise < 70 Gew.-%, insbesondere 10 bis 65 Gew.-% CuO und > 20 Gew.-%, vorzugsweise > 30 Gew.-%, insbesondere 35 bis 90 Gew.-% eines oxidischen Trägers mit sauren Zentren, wobei das Verfahren bei einer Hot-Spot-Temperatur von 240 bis 310°C, vorzugsweise 240 bis 280°C, und Katalysatorbelastungen von 0,01 bis 1,0, vorzugsweise 0,02 bis 1, insbesondere 0,05 bis 0,5 kg Edukt/l Katalysator • Stunde durchgeführt wird.

Unter dem Begriff C₄-Dicarbonsäuren und deren Derivate werden im Bezug auf die vorliegende Anmeldung Maleinsäure und Bernsteinsäure, die gegebenenfalls einen oder mehrere C₁-C₆-Alkylsubstituenten aufweisen sowie die Anhydride dieser gegebenenfalls alkylsubstituierten Säuren verstanden. Ein Beispiel einer solchen Säure ist Citraconsäure. Vorzugsweise werden die jeweiligen Anhydride einer gegebenen Säure eingesetzt. Insbesondere ist das verwendete Edukt MSA.

Das erfindungsgemäße Verfahren zeichnet sich dadurch aus, daß es kostengünstig betrieben werden kann und hohe THF-Ausbeuten und -Selektivitäten erreicht werden. Dies wird durch das Einhalten bestimmter Bedingungen und Parameter erreicht. Weiterhin ist auch eine kontinuierliche Durchführungsweise möglich, die erfindungsgemäß bevorzugt ist.

Ein wichtiger Punkt der vorliegenden Erfindung ist die Wahl des Katalysators, der als katalytisch aktiven Hauptbestandteil Kupferoxid aufweist. Dieses ist auf einem oxidischen Träger, der eine geeignete Anzahl an sauren Zentren besitzen muß, angebracht. Die erforderliche Menge an oxidischem Träger hängt von der darin enthaltenen Menge an sauren Zentren ab. Ein geeignetes Trägermaterial mit einer ausreichenden Zahl an sauren Zentren ist Aluminiumoxid, dessen Verwendung gemäß einer Ausführungsform der vorliegenden Erfindung bevorzugt ist. Gemäß einer anderen Ausführungsform der vorliegenden Erfindung ist es bevorzugt, als saures Trägermaterial eine Kombination von Aluminiumoxid mit Zinkoxid im Gew.-Verhältnis 20:1 bis 1:20, vorzugsweise 5:1 bis 1:5 zu verwenden. Für Materialien, die eine große Menge solcher sauren Zentren aufweisen, liegt die untere Grenze der Menge an aus einem solchen Material bestehendem Träger bei 20 Gew.-%. Die Menge an Kupferoxid liegt bei Werten von <80 Gew.-%. Bevorzugte Katalysatorzusammensetzungen weisen < 70 Gew.-% Kupferoxid und >30 Gew.-% Träger, besonders bevorzugte Katalysatoren 10 bis 65 Gew.-% Kupferoxid und 35 bis 90 Gew.-% Träger auf.

Niedrige Kupferoxid-Gehalte sind auch aufgrund des damit erzielten Kostenvorteils bevorzugt. Durch die sauren Trägermaterialien lassen sich hohe Ausbeuten erzielen.

Optionsweise können die erfindungsgemäß verwendeten Katalysatoren, die Cr-frei sind, ein oder mehrere weitere Metalle oder eine Verbindung davon, vorzugsweise ein Oxid, aus den Gruppen 1 bis 14 (IA bis VIIIA und IB bis IVB der alten IUPAC-Nomenclatur) des Periodensystems der Elemente enthalten. Wird ein solches weiteres Oxid verwendet, wird vorzugsweise TiO₂, ZrO₂, SiO₂ und/oder MgO eingesetzt.

Die eingesetzten Katalysatoren können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und Kupferpulver.

Die Katalysatoren lassen sich nach dem Fachmann bekannten Methoden herstellen. Bevorzugt sind Verfahren, bei denen das Kupferoxid fein verteilt und innig vermischt mit den anderen Bestandteilen anfällt, besonders bevorzugt sind Fällungsreaktionen. Dabei werden in einem Lösungsmittel gelöste Vorläuferverbindungen in Gegenwart weiterer löslicher oder im Lösungsmittel suspendierter Metallverbindungen mit einem Fällungsmittel ausgefällt, abfiltriert, gewaschen, getrocknet und gegebenenfalls calciniert.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Alternativ können erfindungsgemäße Katalysatoren beispielsweise auch durch Aufbringen der Aktivkomponente auf einen Träger hergestellt werden, beispielsweise durch Tränken oder Aufdampfen. Weiterhin können erfindungsgemäße Katalysatoren durch Verformen einer heterogenen Mischung aus Aktivkomponente oder Vorläuferverbindung hiervon mit einer Trägerkomponente oder Vorläuferverbindung hiervon erhalten werden.

Bei der erfindungsgemäßen Hydrierung, bei der neben MSA andere, vorstehend definierte C₄-Dicarbonsäuren oder deren Derivate als Edukt eingesetzt werden können, wird der Katalysator in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in den Reaktor, in dem das erfindungsgemäße Verfahren durchgeführt wird. Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/inertgas-Gemischen bei erhöhten Temperaturen, vorzugsweise zwischen 100 und 300 °C. Bei der sogenannten in-situ-Aktivierung erfolgt die Aktivierung beim Hochfahren der Anlage durch Kontakt mit Wasserstoff bei erhöhter Temperatur.

Die Katalysatoren werden als Formkörper verwendet. Beispiele umfassen Stränge, Rippstränge, andere Extrudatformen, Tabletten, Ringe, Kugeln und Splitt.

Die BET-Oberfläche der Kupferkatalysatoren beträgt im oxidischen Zustand 10 bis 400 m²/g, vorzugsweise 15 bis 200 m²/g, insbesondere 20 bis 150 m²/g. Die Kupferoberfläche (N₂O-Zersetzung) des reduzierten Katalysators beträgt im Einbauzustand > 0,2 m²/g, vorzugsweise > 1 m²/g, insbesondere > 2 m²/g.

Gemäß einer Variante der Erfindung werden Katalysatoren verwendet, die eine definierte Porosität aufweisen. Diese Katalysatoren zeigen als Formkörper ein Porenvolumen von ≥ 0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm. Weiterhin liegt das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %. Oftmals lassen sich durch Verwendung dieser Katalysatoren hohe THF-Ausbeuten und -Selektivitäten erreichen. Die erwähnten Porositäten wurden durch Quecksilber-Intrusion nach DIN 66133 bestimmt. Es wurden die Daten im Porendurchmesserbereich von 4 nm bis 300 µm ausgewertet.

Die erfindungsgemäß verwendeten Katalysatoren besitzen im allgemeinen eine ausreichende Standzeit. Für den Fall, daß die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Ethanol, THF oder GBL, zu waschen und anschließend in einem Gasstrom zu trocknen.

Zum Erzielen der erfindungsgemäßen THF-Selektivitäten ist weiterhin das Einhalten gewisser Reaktionsparameter erforderlich.

Ein wichtiger Parameter ist das Einhalten einer geeigneten Reaktionstemperatur. Dies wird zum einen erreicht durch eine genügend hohe Eingangstemperatur der Edukte. Diese liegt bei Werten von > 220 bis 300 °C, vorzugsweise 235 bis 270 °C. Um eine akzeptable bzw. hohe THF-Selektivität und -Ausbeute zu erhalten, muß die Reaktion so durchgeführt werden, daß am Katalysatorbett, an dem die eigentliche Reaktion stattfindet, eine geeignet hohe Reaktionstemperatur herrscht. Diese sogenannte Hot-Spot-Temperatur wird nach dem Eintritt der Edukte in den Reaktor eingestellt und liegt bei Werten von 240 bis 310°C, vorzugsweise 240 bis 280°C. Das Verfahren wird so durchgeführt, daß die Eingangstemperatur und die Austrittstemperatur der Reaktionsgase unterhalb dieser Hot-Spot-Temperatur liegen. Die Hot-Spot-Temperatur liegt dabei vorteilhafterweise in der 1. Hälfte des Reaktors, insbesondere bei Vorliegen eines Rohrbündelreaktors. Vorzugsweise liegt die Hot-Spot-Temperatur 5 bis 15°C, insbesondere 10 bis 15°C, oberhalb der Eintrittstemperatur. Wenn die Hydrierung unterhalb der Minimaltemperaturen der Eingangs- bzw. Hot-Spot-Temperatur durchgeführt wird, dann steigt im Fall der Verwendung von MSA als Edukt die Menge an GBL bei gleichzeitiger Verminderung der Menge an THF an. Weiterhin ist bei einer solchen Temperatur im Verlauf der Hydrierung eine Desaktivierung des Katalysators durch Belegung mit Bernsteinsäure, Fumarsäure und/oder BSA zu beobachten. Wird dagegen mit MSA als Edukt oberhalb der Maximaltemperaturen der Eingangs- bzw. Hot-Spot-Temperatur hydriert, sinken die THF-Ausbeute und Selektivität auf nicht zufriedenstellende Werte. Es ist hierbei die vermehrte Bildung von n-Butanol und n-Butan zu beobachten, also den Produkten einer weiteren Hydrierung.

Die Katalysatorbelastung der erfindungsgemäßen Hydrierung liegt im Bereich von 0,01 bis 1,0 kg Edukt/l Katalysator • Stunde. Bei einer möglichen, jedoch nicht bevorzugten Rückführung von durch unvollständige Hydrierung entstandenem Zwischenprodukt, im Fall der Verwendung von MSA als Edukt GBL, ist die Katalysatorbelastung die Summe aus frisch zugeführtem Edukt und rückgeführtem Zwischenprodukt. Wird die Katalysatorbelastung über den genannten Bereich hinaus erhöht, ist generell eine Erhöhung des Anteils am Zwischenprodukt im Hydrieraustrag zu beobachten. Vorzugweise liegt die Katalysatorbelastung im Bereich von 0,02 bis 1, insbesondere 0,05 bis 0,5 kg Edukt/l Katalysator • Stunde. Unter dem Begriff "Edukt" wird dabei im Fall der Rückführung auch zunächst entstehendes Hydrierprodukt verstanden, das dann nach Rückführung weiter zu Produkt hydriert wird, also beispielsweise GBL für den Fall des Einsatzes von MSA in die Hydrierreaktion.

Das Wasserstoff/Edukt-Molverhältnis ist ebenfalls ein Parameter, der einen wichtigen Einfluß auf die Produktverteilung und auch die Wirtschaftlichkeit des erfindungsgemäßen Verfahrens hat. Aus wirtschaftlicher Sicht ist ein niedriges Wasserstoff/Edukt-Verhältnis wünschenswert. Die Untergrenze liegt bei einem Wert von 5, wobei jedoch generell höhere Wasserstoff/Edukt-Molverhältnisse von 20 bis 400 angewendet werden. Der Einsatz der oben beschriebenen, erfindungsgemäßen Katalysatoren sowie das Einhalten der oben beschriebenen Temperaturwerte erlaubt den Einsatz günstiger, niedriger Wasserstoff/Edukt-Verhältnisse, die vorzugsweise bei Werten von 20 bis 200, vorzugsweise 40 bis 150 liegen. Der günstigste Bereich liegt bei Werten von 50 bis 100.

Um die erfindungsgemäß verwendeten Wasserstoff/Edukt-Molverhältnisse einzustellen, wird ein Teil, vorteilhafterweise die Hauptmenge, des Wasserstoffs im Kreis gefahren. Hierzu setzt man im allgemeinen die dem Fachmann bekannten Kreisgasverdichter ein. Die chemisch durch die Hydrierung verbrauchte Wasserstoffmenge wird ergänzt. In einer bevorzugten Ausführungsform wird ein Teil des Kreisgases ausgeschleust, um Inertverbindungen, beispielsweise n-Butan, zu entfernen. Der im Kreis geführte Wasserstoff kann auch, gegebenenfalls nach Vorheizen, zum Verdampfen des Eduktstroms benutzt werden.

Auch der Volumenstrom der Reaktionsgase, generell ausgedrückt als GHSV (Gas Hourly Space Velocity) ist eine wichtige Größe des erfindungsgemäßen Verfahrens. Die GHSV-Werte des erfindungsgemäßen Verfahrens liegen bei Werten von 100 bis 10.000 Nm³/m³h, vorzugsweise 1000 bis 3000 Nm³/m³h, insbesondere 1100 bis 2500 Nm³/m³h.

Der Druck, bei dem die erfindungsgemäße Hydrierung durchgeführt wird, liegt bei Werten von 1bis 30 bar, vorzugsweise 2 bis 9 bar, insbesondere 3 bis 7 bar.

Gemeinsam mit dem Wasserstoff-Kreisgas werden alle Produkte im Kreis geführt, die beim Kühlen des aus dem Hydrierreaktor austretenden Gasstroms nicht oder nicht vollständig auskondensieren. Dies sind vor allem THF, Wasser und Nebenprodukte wie Methan und Butan. Die Kühltemperatur beträgt 0 bis 60 °C, vorzugsweise 20 bis 45 °C. Der THF-Gehalt des Kreisgases beträgt 0,1 bis 5 Vol.-%, insbesondere 1 bis 3 Vol.-%.

Aus der Literatur ist bekannt, daß THF und GBL mit Wasserstoff in Gegenwart von Kupfer-Katalysatoren zu n-Butanol hydriert werden. Dabei zeichnet sich das erfindungsgemäße Verfahren dadurch aus, daß trotz der hohen THF-Anteile im Kreisgas, die generell leicht weiter zu n-Butanol hydriert werden, THF-Ausbeuten von über 90%, teilweise auch über 95%, erzielt werden. Als Reaktortypen kommen alle, für heterogen katalysierte Reaktionen mit einem gasförmigen Edukt- und Produktstrom geeigneten Apparate in Betracht. Bevorzugt sind Rohrreaktoren, Schachtreaktoren oder Reaktoren mit innerer Wärmeabfuhr, beispielsweise Rohrbündelreaktoren, es ist auch der Einsatz eines Wirbelbetts möglich. Besonders bevorzugt eingesetzt werden Rohrbündelreaktoren. Es können mehrere Reaktoren parallel oder hintereinander geschaltet eingesetzt werden. Prinzipiell kann zwischen die Katalysatorbetten eine Zwischeneinspeisung erfolgen. Möglich ist auch eine Zwischenkühlung zwischen oder in den Katalysatorbetten. Bei Einsatz von Festbettreaktoren ist eine Verdünnung des Katalysators durch Inertmaterial möglich.

Der aus dem Reaktor austretende Gasstrom wird auf 10 bis 60°C gekühlt. Dabei werden die Reaktionsprodukte auskondensiert und in einen Abscheider geleitet. Der nichtkondensierte Gasstrom wird vom Abscheider abgezogen und dem Kreisgasverdichter zugeführt. Eine kleine Kreisgasmenge wird ausgeschleust. Die auskondensierten Reaktionsprodukte werden dem System kontinuierlich entnommen und der Aufarbeitung zugeführt. Als Nebenprodukte findet man in der auskondensierten Flüssigkeitsphase hauptsächlich n-Butanol neben geringen Mengen an Propanol.

Aus dem Hydrieraustrag werden dann das Azeotrop aus Wasser und gegebenenfalls alkylsubstituiertem THF und gegebenenfalls Nebenprodukt, beispielsweise GBL, durch fraktionierende Destillation voneinander getrennt. Das wasserhaltige THF wird auf an sich bekannte Weise entwässert und destillativ zu spezifikationsgerechtem THF aufgearbeitet. Nebenprodukt wie beispielsweise GBL wird in die Hydrierung zurückgeführt oder destillativ aufgearbeitet.

Das erfindungsgemäße Verfahren zeichnet sich dadurch aus, daß zu hydrierende Edukte unterschiedlicher Reinheit in die Hydrierreaktion eingesetzt werden können. Selbstverständlich kann ein Edukt hoher Reinheit, insbesondere MSA, in die Hydrierreaktion eingesetzt werden. Der erfindungsgemäß verwendete Katalysator sowie die sonstigen erfindungsgemäß gewählten Reaktionsbedingungen ermöglichen aber auch den Einsatz von Edukten, insbesondere MSA, das mit den üblichen, bei der Oxidation von Benzol, Butenen oder n-Butan anfallenden Verbindungen sowie eventuell weiteren Komponenten verunreinigt ist. Somit kann das erfindungsgemäße Hydrierverfahren in einer weiteren Ausführungsform eine vorgeschaltete Stufe umfassen, die das Herstellen des zu hydrierenden Edukts durch partielle Oxidation eines geeigneten Kohlenwasserstoffs sowie das Abtrennen des zu hydrierenden Edukts aus dem damit erhaltenen Produktstrom umfaßt.

Insbesondere ist dieses zu hydrierende Edukt MSA. Dabei wird bevorzugt MSA eingesetzt, welches aus der Partialoxidation von Kohlenwasserstoffen stammt. Geeignete Kohlenwasserstoffströme sind Benzol, C₄-Olefine (z.B. n-Butene, C₄-Raffinatströme) oder n-Butan. Besonders bevorzugt eingesetzt wird n-Butan, da es einen preiswerten, wirtschaftlichen Einsatzstoff darstellt. Verfahren zur Partialoxidation von n-Butan sind beispielsweise in Ullmann's Encyclopedia of Industrial Chemistry, 6^{th} Edition, Electronic Release, Maleic and Fumaric Acids - Maleic Anhydride beschrieben.

Der so erhaltene Reaktionsaustrag wird dann in einem geeigneten organischen Lösungsmittel oder -Gemisch aufgenommen, das bei Atmosphärendruck einen um mindestens 30°C höheren Siedepunkt als MSA hat

Dieses Lösungsmittel (Absorptionsmittel) wird auf eine Temperatur im Bereich zwischen 20 und 160 °C, bevorzugt zwischen 30 und 80 °C, gebracht. Der Maleinsäureanhydrid enthaltende Gasstrom aus der Partialoxidation kann in vielfältiger Weise mit dem Lösungsmittel in Kontakt gebracht werden: (i) Einleiten des Gasstroms in das Lösungsmittel (z.B. über Gaseinleitungsdüsen oder Begasungsringe), (ü) Einsprühen des Lösungsmittels in den Gasstrom und (iii) Gegenstromkontakt zwischen dem nach oben strömenden Gasstrom und dem nach unten strömenden Lösungsmittel in einer Boden- oder Packungskolonne. In allen drei Varianten können die dem Fachmann bekannten Apparate zur Gasabsorption eingesetzt werden. Bei der Wahl des einzusetzenden Lösungsmittels ist darauf zu achten, daß dies nicht mit dem Edukt, beispielsweise dem vorzugsweise eingesetzten MSA, reagiert. Geeignete Lösungsmittel sind: Trikresylphosphat, Dibutylmaleat, hochmolekulare Wachse, aromatische Kohlenwasserstoffe mit einem Molekulargewicht zwischen 150 und 400 und einem Siedepunkt oberhalb 140°C, wie beispielsweise Dibenzylbenzol; Dialkylphthalate mit C₁-C₈-Alkylgruppen, beispielsweise Dimethylphthalat, Diethylphthalat, Dibutylphthalat, Di-n-Propyl-und Di-iso-Propylphthalat; Mono-, Di-, Tri- und Tetraester von Cyclohexandi-, tri- und tetrasäuren, wobei es sich bei den Estern um Alkyl-, Cycloalkyl-, Hydroxy- und Alkoxyalkylester handelt und diese Gruppen 1 bis 30, vorzugsweise 2 bis 20, insbesondere 3 bis 18 Kohlenstoffatome aufweisen und - im Fall nicht cyclischer Gruppen - linear oder verzweigt sein können; Beispiele umfassen: Cyclohexan-1,4-dicarbonsäuredialkylester mit identischen Alkoholresten, Cyclohexan-1,3-dicarbonsäuredialkylester mit identischen Alkoholresten, Cyclohexan-1,2-dicarbonsäuredialkylester mit identischen Alkoholresten, gemischte Ester der Cyclohexan-1,2-dicarbonsäure mit C1 bis C13-Alkoholen, gemischte Ester der Cyclohexan-1,3-dicarbonsäure mit C1 bis C13-Alkoholen, gemischte Ester der Cyclohexan-1,4-dicarbonsäure mit C1 bis C13-Alkoholen, Cyclohexan-1,2,4-tricarbonsäurealkylester, Cyclohexan-1,3,5-tricarbonsäurealkylester, Cyclohexan-1,2,3-tricarbonsäurealkylester, Cyclohexan-1,2,4,5-tetracarbonsäurealkylester; Mono-, Di-, Triund Tetraester von Cyclohexendi-, tri- und tetrasäuren, wobei es sich bei den Estern um Alkyl-, Cycloalkyl-, Hydroxy- und Alkoxyalkylester handelt und diese Gruppen 1 bis 30, vorzugsweise 2 bis 20, insbesondere 3 bis 18 Kohlenstoffatome aufweisen und - im Fall nicht cyclischer Gruppen - linear oder verzweigt sein können; Beispiele umfassen: Cyclohexen-1,4-dicarbonsäuredialkylester mit identischen Alkoholresten, Cyclohexen-1,3-dicarbonsäuredialkylester mit identischen Alkoholresten, Cyclohexen-1,2-dicarbonsäuredialkylester mit identischen Alkoholresten, gemischte Ester der Cyclohexen-1,2-dicarbonsäure mit C1 bis C13-Alkoholen, gemischte Ester der Cyclohexen-1,3-dicarbonsäure mit C1 bis C13-Alkoholen, gemischte Ester der Cyclohexen-1,4-dicarbonsäure mit C1 bis C13-Alkoholen, Cyclohexen-1,2,4-tricarbonsäurealkylester, Cyclohexen-1,3,5-tricarbonsäurealkylester, Cyclohexen-1,2,3-tricarbonsäurealkylester, Cyclohexen-1,2,4,5-tetracarbonsäurealkylester; Di-C₁-C₄-Alkylester anderer aromatischer und aliphatischer Dicarbonsäuren, beispielsweise Dimethyl-2,3-Naphthalin-Dicarbonsäure, Methylester langkettiger Fettsäuren mit beispielsweise 14 bis 30 Kohlenstoffatomen, hochsiedende Ether, beispielsweise Dimethylether von Polyethylenglykol, beispielsweise Tetraethylenglykoldimethylether.

Der Einsatz von Phthalaten ist bevorzugt.

Die nach der Behandlung mit dem Absorptionsmittel resultierende Lösung hat generell einen MSA-Gehalt von etwa 5 bis 400 Gramm pro Liter.

Der nach der Behandlung mit dem Absorptionsmittel verbleibende Abgasstrom enthält hauptsächlich die Nebenprodukte der vorangegangenen Partialoxidation, wie Wasser, Kohlenmonoxid, Kohlendioxid, nicht umgesetzte Butane, Essig- und Acrylsäure. Der Abgasstrom ist praktisch frei von MSA.

Anschließend wird das gelöste MSA aus dem Absorptionsmittel ausgetrieben. Dies erfolgt mit Wasserstoff bei oder maximal 10% oberhalb des Druckes der anschließenden Hydrierung oder alternativ im Vakuum mit anschließender Kondensation von verbleibendem MSA. In der Strippkolonne wird ein Temperaturprofil beobachtet, das sich aus den Siedepunkten von MSA am Kopf und dem nahezu MSA-freien Absorptionsmittel am Sumpf der Kolonne bei dem jeweiligen Kolonnendruck und der eingestellten Verdünnung mit Trägergas (im ersten Fall mit Wasserstoff) ergibt. Im Fall der Direktstrippung mit Wasserstoff wird bei einer Kopftemperatur von 130°C und einem Druck von 5 bar gearbeitet.

Um Verluste an Lösungsmittel zu verhindern, können sich oberhalb der Zufuhr des Roh-MSA-Stromes Rektifiziereinbauten befinden. Das vom Sumpf abgezogene, nahezu MSAfreie Absorptionsmittel wird wieder der Absorptionszone zugeführt. Im Fall der Direktstrippung mit Wasserstoff wird vom Kopf der Kolonne ein nahezu gesättigter Gasstrom von MSA in Wasserstoff mit einer Temperatur von 180 °C und einem Druck von 5 bar abgezogen. Das H₂/MSA-Verhältnis liegt bei etwa 20 bis 400. Im anderen Fall wird das kondensierte MSA in einen Verdampfer gepumpt und dort in den Kreisgasstrom verdampft.

Der MSA-Wasserstoff-Strom enthält noch Nebenprodukte, die bei der partiellen Oxidation von n-Butan, Butenen oder Benzol mit Sauerstoff enthaltenden Gasen entstehen, sowie nicht abgetrenntes Absorptionsmittel. Hierbei handelt es sich vor allem um Essigsäure und Acrylsäure als Nebenprodukte, Wasser, Maleinsäure sowie die vorzugsweise als Absorptionsmittel verwendeten Dialkylphthalate. Das MSA enthält Essigsäure in Mengen von 0,01 bis 1 Gew.-%, vorzugsweise 0, 1 bis 0,8 Gew.-% und Acrylsäure in Mengen von 0,01 bis 1 Gew.-%, vorzugsweise 0,1 bis 0,8 Gew.-%, bezogen auf MSA. In der Hydrierstufe werden Essigsäure und Acrylsäure ganz oder teilweise zu Ethanol bzw. Propanol hydriert. Der Maleinsäure-Gehalt beträgt 0,01 bis 1 Gew.-%, insbesondere 0,05 bis 0,3 Gew.-%, bezogen auf MSA.

Werden Dialkylphthalate als Absorptionsmittel eingesetzt, hängt deren Gehalt im MSA stark vom richtigen Betrieb der Strippkolonne, insbesondere vom Verstärkungsteil ab. Phthalatgehalte von bis 1,0 Gew.-% , insbesondere bis 0,5 Gew.-% sollten bei geeigneter Betriebsweise nicht überschritten werden, da sonst der Verbrauch an Absorptionsmittel zu hoch wird.

Der so erhaltene Wasserstoff/Maleinsäureanhydrid-Strom wird nun der Hydrierzone zugeführt und wie oben beschrieben hydriert. Die Katalysatoraktivität und -standzeit ist dabei verglichen mit dem Einsatz von stark, beispielsweise durch Destillation, vorgereinigtem MSA praktisch unverändert. Das erfindungsgemäße Verfahren erlaubt THF-Ausbeuten, die bei Werten von etwa 90%, günstigenfalls etwa 95%, liegen. Es wird dabei auch eine hohe Produktselektivität erzielt. GBL wird zumeist in Mengen unter 5% gebildet.
Das erfindungsgemäße Verfahren wird nun in den nachfolgenden Beispielen näher erläutert.

### Beispiele

### Beispiel 1

### a) Katalysatorherstellung

In einem beheizbaren und mit Rührwerk ausgestatteten Fälltopf werden 1,5 l Wasser vorgelegt und auf 80 °C erwärmt. In dieses Fällgefäß werden im Verlauf einer Stunde eine Metallsalzlösung bestehend aus 731 g Cu(NO₃)₂*2,5 H₂O und 1840 g Al(NO₃)₃*9 H₂O in 2000 ml Wasser und gleichzeitig eine 20 Gew.-% Sodalösung unter Rühren zudosiert, bis im Fällgefäß ein pH-Wert von 8 erreicht ist und weitere 15 min bei diesem pH-Wert gerührt. Der Gesamtverbrauch an Sodalösung liegt bei 5,6 kg. Die gebildete Suspension wird abfiltriert und mit Wasser gewaschen, bis das ablaufende Waschwasser kein Nitrat (< 25 ppm) mehr enthält. Der Filterkuchen wird zunächst bei 120°C getrocknet und anschließend bei 600 °C calciniert. Der so hergestellte Katalysator enthält 50 Gew.-% CuO und 50 Gew.-% Al₂O₃. 400 g dieses Katalysatorpulvers werden auf eine Korngröße von < 1mm zerkleinert, mit 12 g Graphitpulver versetzt, intensiv durchmischt und zu Tabletten von 3 mm Durchmesser und 3 mm Höhe verpresst.

### b) Katalysator-Aktivierung

Vor dem Reaktionsbeginn wird der Katalysator in der Hydrierapparatur einer Wasserstoffbehandlung unterzogen. Hierzu wird der Reaktor auf 180 °C temperiert und der Katalysator die in Tabelle 1 angegebene Zeit mit dem jeweils angegebenen Gemisch aus Wasserstoff und Stickstoff bei Atmosphärendruck aktiviert.

**Tabelle 1**

| Zeit (Minuten) | Wasserstoff (Nl/h) | Stickstoff (Nl/h) |
|---|---|---|
| 120 | 10 | 550 |
| 30 | 25 | 400 |
| 15 | 60 | 100 |
| 180 | 60 | 0 |

### c) Hydrierapparatur

Die zur Hydrierung verwendete Druckapparatur besteht aus einem Verdampfer, einem Reaktor, einem Kühler mit Quenchzulauf, einer Wasserstoffzufuhr, einer Abgasleitung und einem Kreisgasgebläse. Der Druck in der Apparatur wird konstant gehalten.

Das aufgeschmolzene MSA wird von oben auf den vorgeheizten (245 °C) Verdampfer gepumpt und verdampft. Auf den Verdampfer gelangt ebenfalls von oben eine Mischung aus frischem Wasserstoff und Kreisgas. Wasserstoff und MSA gelangen so von unten in den temperierten Reaktor. Der Reaktorinhalt besteht aus einem Gemisch aus Glasringen und Katalysator. Nach der Hydrierung verläßt das entstandene THF zusammen mit Wasser, anderen Reaktionsprodukten und Wasserstoff den Reaktor und wird im Kühler durch Quenchen niedergeschlagen. Ein Teil des Kreisgases wird ausgeschleust, bevor der Rest, mit Frischwasserstoff vermischt, wieder in den Verdampfer eintritt.

Der kondensierte flüssige Reaktionsaustrag, das Abgas und das Kreisgas werden gaschromatographisch quantitativ analysiert.

### Beispiel 1d

### d) Hydrierung von aus n-Butan hergestelltem Maleinsäureanhydrid

Der Reaktor der in Beispiel 1b beschriebenen Hydrierapparatur wird mit 220 ml des nach Beispiel 1 a hergestellten Katalysators und 130 ml Glasringen gefüllt. Die Aktivierung erfolgte wie in Beispiel 1 b beschrieben.

Als Edukt wird aus n-Butan hergestelltes Maleinsäureanhydrid eingesetzt, das 500 ppm Acrylsäure, 1500 ppm Essigsäure und 100 ppm Dibutylphthalat enthält. Die Umsetzung wird 1000 h lang durchgeführt. Dabei wird über den gesamten Zeitraum keinerlei Desaktivierung des Katalysators, d.h. kein Rückgang des Maleinsäureanhydrid-Umsatzes und/oder der Tetrahydrofuran-Ausbeute beobachtet. Butandiol wird gaschromatographisch nicht beobachtet. In Tabelle 2 sind die Reaktionsparameter der Hydrierung und die Ergebnisse zusammengefaßt.

Nach fraktionierender Destillation der Hydrierausträge wird Tetrahydrofuran mit einer Reinheit von 99,96% isoliert. Dieses THF erfüllt die Spezifikation für die Verwendung als Ausgangsprodukt für Poly-THF.

### Beispiel 1e

Anstelle von 50 Gew.-% CuO und 50 Gew.-% Al₂O₃ wird ein Katalysator mit der Zusammensetung 40 Gew.-% CuO, 40 Gew.-% ZnO, und 20 Gew.-% Al₂O₃ verwendet. Auch er wird nach Aktivierung des Katalysators mit dem in Beispiel 1 d beschriebenen Maleinsäureanhydrid 1000 h lang ohne Desaktivierung betrieben. Butandiol wird gaschromatographisch nicht beobachtet. In Tabelle 2 sind die Reaktionsparameter der Hydrierung und die Ergebnisse zusammengefaßt.

Nach fraktionierender Destillation der Hydrierausträge wird Tetrahydrofuran mit einer Reinheit von 99,94 % isoliert.

**Tabelle 2**

| Beispiel | Temp. (°C) | Druck (bar) | GHSV (h⁻¹) | Kat.- Belastung (kg/l·h) | Molverh. H₂:MSA | Umsatz³⁾ (%) | Mol-% | | | |
|---|---|---|---|---|---|---|---|---|---|---|
| | | | | | | | THF | GBL | n-BuOH | n-Butan |
| 1d¹⁾ | 255- 258 | 5,1 | 2000 | 0,1 0,1 | 85:1 | 100 | 87-88 | 4-5 | 6 | 0,1 |
| 1e²⁾ | | | 2000 | | | | 87 | 3 | 5 | 0,1 |

| | | | | | | | | | | |
|---|---|---|---|---|---|---|---|---|---|---|
| n-BuOH = n-Butanol ¹⁾ Katalysator: 50 Gew.-% CuO, 50 Gew.-% Al₂O₃ | | | | | | | | | | |
| ²⁾ Katalysator: 40 Gew.-% CuO, 40 Gew.-% ZnO, 20 Gew.-% Al₂O₃ | | | | | | | | | | |
| ³⁾ Gesamtumsatz von MSA und Bernsteinsäureanhydrid (BSA) | | | | | | | | | | |

Die Ergebnisse der Beispiele 1d und 1e zeigen, daß man bei der kontinuierlichen Hydrierung von Acrylsäure, Essigsäure und Dibutylphthalat enthaltendem Maleinsäureanhydrid in Gegenwart von Cu/Al₂O₃- und Cu/ZnO/Al₂O₃-Katalysatoren über lange Reaktionszeiten hinweg unverändert hohe Katalysator-Aktivität bei unverändert hohen Tetrahydrofuran-Ausbeuten erzielt. Außerdem lassen sich die Hydrierausträge destillativ zu Tetrahydrofuran hoher Reinheit aufarbeiten, welches die geforderte Tetrahydrofuranspezifikation erfüllt.

### Beispiel 2

Ein analog zu Beispiel 1a hergestellter Katalysator, der zu 60% aus Kupferoxid und zu 40% aus Aluminiumoxid besteht, wird in die oben beschriebene Hydrierapparatur eingebaut und wie in Beispiel 1b beschrieben mit Wasserstoff vorbehandelt. Als Edukt wird aus n-Butan hergestelltes Maleinsäureanhydrid, welches 5000 ppm Acrylsäure, 1500 ppm Essigsäure und 100 ppm Dibutylphthalat enthält, eingesetzt. Die Reaktion wird bei einem Druck von 5 bar durchgeführt, alle anderen Reaktionsparameter und Ergebnisse sind in Tabelle 3 dargestellt. Bei den Beispielen 2a, 2b und 2d handelt es sich dabei um Vergleichsversuche, die bei Reaktionsbedingungen durchgeführt wurden, die außerhalb der erfindungsgemäßen Parameter liegen.

**Tabelle 3**

| Beispiel | Temp. (°C) | Druck (bar) | GHSV (h⁻¹⁾ | Kat.-Bel. (kg/l·h) | Mol-Verh. H₂:MSA | (Mol%) | | | |
|---|---|---|---|---|---|---|---|---|---|
| | | | | | | THF | GBL | n-BuOH | Butan |
| 2a¹⁾ | 235 | 5 | 5000 | 0.04 | 550:1 | 86,0 | 2,0 | 9,0 | 1,0 |
| 2b¹⁾ | | | 2700 | 0.12 | 100:1 | 58,6 | 36,7 | 2.3 | 0,3 |
| 2c | 255 | | 2700 | 0.13 | 90:1 | 89.4 | 2.6 | 7.0 | 1.0 |
| 2d¹⁾ | 285 | | 2700 | 0.13 | 90:1 | 65.3 | - | 12.5 | 19.6 |

| | | | | | | | | | |
|---|---|---|---|---|---|---|---|---|---|
| ¹⁾ Vergleichsbeispiele | | | | | | | | | |

Das Ergebnis aus Beispiel 2c zeigt, daß man bei 255°C hohe Tetrahydrofuran-Ausbeuten erzielt. Senkt man nach Beispiel 2b die Temperatur auf 235°C ab, so steigt die Butyrolacton-Ausbeute auf 36,7%. Erst durch Senkung der Katalysator-Belastung auf ein Drittel und Erhöhung des Wasserstoff/Maleinsäureanhydrid-Verhältnisses (Beispiel 2a) nähert man sich den Tetrahydrofuran-Ausbeuten von Beispiel 2c an. Steigert man (nach Beispiel 2d) die Hydriertemperatur auf 285°C, so sinkt die Tetrahydrofuran-Ausbeute auf 65,3%.

### Beispiel 3

Beispiel 2c wird bei einer GHSV von 1800 h⁻¹ unter ansonsten gleichen Bedingungen mit einem Katalysator wiederholt, der 60 % CuO und 40 % Al₂O₃ enthält. Die Tetrahydrofuran-Ausbeute beträgt 93 %. Butyrolacton wird nicht beobachtet.

### Beispiel 4: (Vergleichsbeispiel)

Beispiel 3 der JP 2-233 631 wird nachgearbeitet: Hierzu wird der in Beispiel 3 beschriebene Cu/Al₂O₃-Katalysator nach der dort angegebenen Vorschrift hergestellt. Dann wird das Rein-MSA/GBL-Gemisch unter den angegebenen Bedingungen hydriert. Man findet eine THF-Ausbeute von 90 % und eine GBL-Ausbeute von 7 %, weiterhin 1,9 % n-Butanol.

Beim Wechsel von MSA/GBL (Molverhältnis 1:3) zu Rein-MSA (Rein-MSA-Molmenge entspricht der Summe der Molmengen an MSA +GBL, Katalysator-Belastung 0,03 kg MSA/l Katalysator • Stunde) als Feed sinkt die THF-Ausbeute nach 10 Stunden auf 12 % THF und 59 % GBL. Außerdem entstehen 28 % Maleinsäure und Bernsteinsäure (bezogen auf eingesetztes MSA) (Tabelle 4). Im Austragsteil der Hydrierapparatur und im Kreisgassystem lagerten sich große Mengen an Dicarbonsäuregemisch ab.

**Tabelle 4**

| Beispiel | MSA-Ums. (%) | (Mol-%)²⁾ | | | | |
|---|---|---|---|---|---|---|
| 4¹⁾ | ca. 95 | THF | GBL | BDO³⁾ | n-BuOH | n-Butan |
| | | 12 | 59 | - | 0,6 | - |

| | | | | | | |
|---|---|---|---|---|---|---|
| ¹⁾ Vergleichsbeispiel | | | | | | |
| ²⁾ Bezogen auf eingesetztes MSA | | | | | | |
| ³⁾ Butandiol-1,4 | | | | | | |

Die Ergebnisse zeigen, daß man unter den Bedingungen von Beispiel 3 in JP 2-233 631 bei Verwendung von MSA anstelle von MSA/GBL-Gemischen nur sehr niedrige THF-Ausbeuten erzielt. Trotz niedriger Katalysator-Belastungen kommt es in der Hydrieranlage zu Verstopfungen durch Feststoffe (Dicarbonsäuregemische), die eine Durchführung der MSA-Hydrierung in technischem Maßstab unmöglich machen.

## Patentansprüche

1. Verfahren zur Herstellung von gegebenenfalls alkylsubstituiertem THF durch katalytische Hydrierung in der Gasphase von C₄-Dicarbonsäuren und/oder deren Derivaten mit einem Katalysator enthaltend <80 Gew.-%, vorzugsweise < 70 Gew.-%, insbesondere 10 bis 65 Gew.-% CuO und >20 Gew.-%, vorzugsweise > 30 Gew.-%, insbesondere 35 bis 90 Gew:-% eines oxidischen Trägers mit sauren Zentren, wobei das Verfahren bei einer Hot-Spot-Temperatur von 240 bis 310 °C, vorzugsweise 240 bis 280 °C, und Katalysatorbelastungen von 0,01 bis 1,0, vorzugsweise 0,02 bis 1, insbesondere 0,05 bis 0,5 kg Edukt/l Katalysator • Stunde durchgerührt wird.

2. Verfahren nach Anspruch 1, **dadurch gekennzeichnet, daß** der oxidische Träger Al₂O₃ oder eine Kombination aus Al₂O₃/ZnO im Gew.-Verhältnis 20:1 bis 1:20, vorzugsweise 5:1 bis 1:5 ist.

3. Verfahren nach Anspruch 1 und 2, **dadurch gekennzeichnet, daß** dieses bei Drücken von 1 bis 30 bar, vorzugsweise 2 bis 9 bar, insbesondere 3 bis 7 bar, durchgeführt wird.

4. Verfahren nach einem der Ansprüche 1 bis 3, **dadurch gekennzeichnet, daß** das Molverhältnis Wasserstoff/Edukt bei Werten von 20 bis 400, vorzugsweise 20 bis 200, insbesondere 40 bis 150, meistbevorzugt 50 bis 100 liegt

5. Verfahren nach einem der Ansprüche 1 bis 4, **dadurch gekennzeichnet, daß** die GHSV bei Werten von 100 bis 10.000 Nm³/m³h, vorzugsweise 1000 bis 3000 Nm³/m³h, insbesondere 1100 bis 2500 Nm³/m³h liegt.

6. Verfahren nach einem der Ansprüche 1 bis 5, **dadurch gekennzeichnet, daß** die Eingangstemperatur bei Werten von > 220 bis 300°C, vorzugsweise 235 bis 270°C und ca. 5 bis 15°C, vorzugsweise ca. 10 bis 15°C, unterhalb der Hot-Spot-Temperatur liegt.

7. Verfahren nach einem der Ansprüche 1 bis 6, **dadurch gekennzeichnet, daß** die Hot-Spot-Temperatur in der ersten Hälfte des Reaktors liegt.

8. Verfahren nach einem der Ansprüche 1 bis 7, **dadurch gekennzeichnet, daß** ein oder mehrere weitere Metalle oder eine Verbindung davon, vorzugsweise ein Oxid, aus der Gruppe bestehend aus den Elementen der Gruppen 1 bis 14 des Periodensystems der Elemente in dem Katalysator vorhanden sind, vorzugsweise eine Substanz aus der Gruppe bestehend aus ZrO₂, TiO₂, SiO₂ und MgO.

9. Verfahren nach einem der Ansprüche 1 bis 8, **dadurch gekennzeichnet, daß** der Katalysator vor oder nach dem Einbau in den Reaktor und vor dem Einsatz in die Hydrierreaktion durch Reduktion aktiviert wird, vorzugsweise durch Behandlung mit Wasserstoff oder einem Wasserstoff/Inertgas-Gemisch.

10. Verfahren nach einem der Ansprüche 1 bis 9, **dadurch gekennzeichnet, daß** der Katalysator ein Hilfsmittel in einer Menge < 10 Gew.-%, vorzugsweise Graphit, Stearinsäure, Kieselgel und/oder Kupferpulver, enthält.

11. Verfahren nach einem der Ansprüche 1 bis 10, **dadurch gekennzeichnet, daß** der Katalysator-Formkörper ein Porenvolumen von ≥ 0,01 ml/g für Porendurchmesser > 50 nm, vorzugsweise ≥ 0,025 ml/g für Porenduchmesser > 100 nm, insbesondere ≥ 0,05 ml/g für Porendurchmesser > 200 nm aufweist.

12. Verfahren nach einem der Ansprüche 1 bis 11, **dadurch gekennzeichnet, daß** in dem Katalysator-Formkörper das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %, liegt.

13. Verfahren nach einem der Ansprüche 1 bis 12, **dadurch gekennzeichnet, daß** ein Festbettreaktor, vorzugsweise ein Rohrreaktor, ein Schachtreaktor, ein Wirbelbettreaktor oder ein Reaktor mit innerer Wärmeabfuhr, insbesondere ein Rohrbündelreaktor, eingesetzt wird.

14. Verfahren nach einem der Ansprüche 1 bis 13, **dadurch gekennzeichnet, daß** Maleinsäureanhydrid als Edukt in die Reaktion eingesetzt wird.

15. Verfahren nach einem der Ansprüche 1 bis 14, **dadurch gekennzeichnet, daß** Maleinsäureanhydrid eingesetzt wird, das durch Oxidation von Benzol, C₄-Olefinen oder n-Butan hergestellt wurde, wobei das durch Oxidation erhaltene Roh-Maleinsäureanhydrid mit einem Lösungsmittel aus dem Rohproduktgemisch extrahiert und anschließend aus diesem Lösungsmittel mit Wasserstoff ausgetrieben wurde.

16. Verfahren nach einem der Ansprüche 1 bis 15, **dadurch gekennzeichnet, daß** das Absorptionsmittel ausgewählt ist aus der Gruppe bestehend aus Trikresylphosphat, Dibutylmaleat, hochmolekularen Wachsen, aromatischem Kohlenwasserstoff mit einem Molekulargewicht zwischen 150 und 400 und einem Siedepunkt oberhalb 140 °C, vorzugsweise Dibenzylbenzol, Di-C₁-C₄-Alkylestern aromatischer und aliphatischer Dicarbonsäuren, vorzugsweise Dimethyl-2,3-Naphthalin-Dicarbonsäure und/oder Dimethyl-1,4-Cyclohexan-Dicarbonsäure, Methylestern langkettiger Fettsäuren mit 14 bis 30 Kohlenstoffatomen, hochsiedenden Ethern, vorzugsweise Dimethylether von Polyethylenglykol, vorzugsweise von Tetraethylenglykol, und Dialkylphthalaten mit C₁-C₈-Alkylgruppen, vorzugsweise aus der Gruppe bestehend aus Dialkylphtalaten mit C₁-C₈-Alkylgruppen, insbesondere Dimethylphthalat, Diethylphthalat, Di-n-Propyl-Phthalat, Di-iso-Butyl-Phthalat und Dibutylphthalat.

17. Verfahren nach einem der Ansprüche 1 bis 16, **dadurch gekennzeichnet, daß** das Maleinsäureanhydrid aus dem Absorptionsmittel im Vakuum oder bei Drücken, die dem Druck der Hydrierung entsprechen oder maximal 10 % oberhalb dieses Druckes liegen, ausgetrieben wird.

## Claims

1. A process for preparing unsubstituted or alkylsubstituted THF by catalytic hydrogenation in the gas phase of C₄-dicarboxylic acids and/or their derivatives using a catalyst comprising < 80% by weight, preferably < 70% by weight, in particular from 10 to 65% by weight, of CuO and > 20% by weight, preferably > 30% by weight, in particular from 35 to 90% by weight, of an oxidic support having acid centers, at a hot spot temperature of from 240 to 310°C, preferably from 240 to 280°C, and a WHSV over the catalyst of from 0.01 to 1.0, preferably from 0.02 to 1, in particular from 0.05 to 0.5, kg of starting material/l of catalyst x hour.

2. A process as claimed in claim 1, wherein the oxidic support is Al₂O₃ or a combination of Al₂O₃/ZnO in a weight ratio of from 20:1 to 1:20, preferably from 5:1 to 1:5.

3. A process as claimed in claim 1 or 2 carried out at pressures of from 1 to 30 bar, preferably from 2 to 9 bar, in particular from 3 to 7 bar.

4. A process as claimed in any of claims 1 to 3, wherein the molar ratio of hydrogen/starting material is from 20 to 400, preferably from 20 to 200, in particular from 40 to 150, most preferably from 50 to 100.

5. A process as claimed in any of claims 1 to 4, wherein the GHSV is from 100 to 10,000 Standard m³/m³h, preferably from 1000 to 3000 Standard m³/m³h, in particular from 1100 to 2500 Standard m³/m³h.

6. A process as claimed in any of claims 1 to 5, wherein the inlet temperature is from > 220 to 300°C, preferably from 235 to 270°C, and is from about 5 to 15°C, preferably from about 10 to 15°C, below the hot spot temperature.

7. A process as claimed in any of claims 1 to 6, wherein the hot spot is located in the first half of the reactor.

8. A process as claimed in any of claims 1 to 7, wherein one or more further metals or compounds thereof, preferably oxides, from the group consisting of the elements of groups 1 to 14 of the Periodic Table of the Elements are present in the catalyst, preferably a substance selected from the group consisting of ZrO₂, TiO₂, SiO₂ and MgO.

9. A process as claimed in any of claims 1 to 8, wherein the catalyst is activated by reduction, preferably by treatment with hydrogen or a hydrogen/inert gas mixture, before or after installation in the reactor and before use in the hydrogenation reaction.

10. A process as claimed in any of claims 1 to 9, wherein the catalyst further comprises an auxiliary in an amount of < 10% by weight, preferably graphite, stearic acid, silica gel and/or copper powder.

11. A process as claimed in any of claims 1 to 10, wherein the shaped catalyst body has a pore volume of ≥ 0.01 ml/g for pore diameters of > 50 nm, preferably ≥ 0.025 ml/g for pore diameters of > 100 nm, in particular ≥ 0.05 ml/g for pore diameters of > 200 nm.

12. A process as claimed in any of claims 1 to 11, wherein the ratio of macropores having a diameter of > 50 nm to the total pore volume for pores having a diameter of > 4 nm in the shaped catalyst body is > 10%, preferably > 20%, in particular > 30%.

13. A process as claimed in any of claims 1 to 12, wherein a fixed-bed reactor, preferably a tube reactor, a shaft reactor, a fluidized-bed reactor or a reactor with internal heat removal, in particular a shell-and-tube reactor, is used.

14. A process as claimed in any of claims 1 to 13, wherein maleic anhydride is used as starting material for the reaction.

15. A process as claimed in any of claims 1 to 14, wherein maleic anhydride prepared by oxidation of benzene, C₄-olefins or n-butane, where the crude maleic anhydride obtained by oxidation has been extracted from the crude product mixture using a solvent and has subsequently been stripped from this solvent by means of hydrogen, is used.

16. A process as claimed in any of claims 1 to 15, wherein the absorption medium is selected from the group consisting of tricresyl phosphate, dibutyl maleate, high molecular weight waxes, aromatic hydrocarbons having a molecular weight in the range from 150 to 400 and a boiling point above 140°C, preferably dibenzylbenzene, di-C₁-C₄-alkyl esters of aromatic and aliphatic dicarboxylic acids, preferably dimethyl naphthalene-2,3-dicarboxylate and/or dimethyl cyclohexane-1,4-dicarboxylate, methyl esters of long-chain fatty acids having from 14 to 30 carbon atoms, high-boiling ethers, preferably dimethyl ethers of polyethylene glycols, preferably of tetraethylene glycol, and dialkyl phthalates having C₁-C₈-alkyl groups, preferably from the group consisting of dialkyl phthalates having C₁-C₈-alkyl groups, in particular dimethyl phthalate, diethyl phthalate, di-n-propyl phthalate, diisobutyl phthalate and dibutyl phthalate.

17. A process as claimed in any of claims 1 to 16, wherein the maleic anhydride is stripped from the absorption medium under reduced pressure or at pressures which correspond to the pressure in the hydrogenation or are at most 10% above this pressure.

## Revendications

1. Procédé de préparation de THF le cas échéant substitué par alkyle, par hydrogénation catalytique en phase gazeuse d'acides dicarboxyliques en C₄ et/ou de leurs dérivés, avec un catalyseur contenant < 80% en poids, de préférence < 70% en poids, en particulier 10 à 65% en poids de CuO et > 20% en poids, de préférence > 30% en poids, en particulier de 35 à 90% en poids d'un support oxyde avec des centres acides, où le procédé est réalisé à une température Hot-Spot de 240 à 310°C, de préférence de 240 à 280°C et des charges de catalyseur allant de 0,01 à 1,0, de préférence 0,02 à 1, en particulier de 0,05 à 0,5 kg de réactif/litre de catalyseur.heure.

2. Procédé selon la revendication 1, **caractérisé en ce que** le support oxyde est Al2O3 ou une combinaison Al₂O₃/ZnO en un rapport pondéral de 20:1 à 1:20, de préférence de 5:1 à 1:5.

3. Procédé selon la revendication 1 ou 2, **caractérisé en ce que** celui-ci est réalisé à des pressions allant de 1 à 30 bar, de préférence de 2 à 9 bar, en particulier de 3 à 7 bar.

4. Procédé selon l'une quelconque des revendications 1 à 3, **caractérisé en ce que** le rapport molaire hydrogène/réactif se situe dans l'intervalle allant de 20 à 400, de préférence de 20 à 200, en particulier de 40 à 150, de manière la plus préférée de 50 à 100.

5. Procédé selon l'une quelconque des revendications 1 à 4, **caractérisé en ce que** le GHSV se situe dans l'intervalle allant de 100 à 10 000 Nm3/m3h, de préférence de 1000 à 3000 Nm3/m3h, en particulier de 1100 à 2500 Nm3/m3h.

6. Procédé selon l'une quelconque des revendications 1 à 5, **caractérisé en ce que** la température d'entrée se situe dans l'intervalle allant de > 220 à 300°C, de préférence de 235 à 270°C et d'environ 5 à 15°C, de préférence d'environ 10 à 15°C, sous la température Hot-Spot.

7. Procédé selon l'une quelconque des revendications 1 à 6, **caractérisé en ce que** la température Hot-Spot se situe dans la première moitié du réacteur.

8. Procédé selon l'une quelconque des revendications 1 à 7, **caractérisé en ce qu'**un ou plusieurs métaux ou un composé de ceux-ci, de préférence un oxyde, du groupe consistant en les éléments des groupes 1 à 14 du système périodique des éléments, sont présents dans le catalyseur, de préférence une substance du groupe consistant en ZrO₂, TiO₂, SiO₂ et MgO.

9. Procédé selon l'une quelconque des revendications 1 à 8, **caractérisé en ce que** le catalyseur est activé par réduction avant ou après l'introduction dans le réacteur et avant la mise en oeuvre dans la réaction d'hydrogénation, de préférence par traitement avec de l'hydrogène ou un mélange hydrogène/gaz inerte.

10. Procédé selon l'une quelconque des revendications 1 à 9, **caractérisé en ce que** le catalyseur contient un auxiliaire en une quantité < 10% en poids, de préférence du graphite, de l'acide stéarique, du gel de silice et/ou de la poudre de cuivre.

11. Procédé selon l'une quelconque des revendications 1 à 10, **caractérisé en ce que** le corps moulé de catalyseur présente un volume des pores de ≥ 0,01 ml/g pour un diamètre des pores > 50 nm, de préférence de ≥ 0,025 ml/g pour un diamètre des pores > 100 nm, en particulier de ≥ 0,05 ml/g pour un diamètre des pores > 200 nm.

12. Procédé selon l'une quelconque des revendications 1 à 11, **caractérisé en ce que** dans le corps moulé de catalyseur, le rapport des macropores avec un diamètre > 50 nm aux volume total des pores avec un diamètre > 4 nm se situe à des valeurs > 10%, de préférence > 20%, en particulier > 30%.

13. Procédé selon l'une quelconque des revendications 1 à 12, **caractérisé en ce que** l'on met en oeuvre un réacteur à lit fixe, de préférence un réacteur tubulaire, un réacteur à puits, un réacteur à lit fluidisé ou un réacteur avec élimination interne de la chaleur, en particulier un réacteur à faisceau tubulaire.

14. Procédé selon l'une quelconque des revendications 1 à 13, **caractérisé en ce que** l'on met en oeuvre dans la réaction, l'anhydride maléique comme réactif.

15. Procédé selon l'une quelconque des revendications 1 à 14, **caractérisé en ce que** l'on met en oeuvre l'anhydride maléique, qui est préparé par oxydation de benzène, d'oléfines en C₄ ou de n-butane, où l'anhydride maléique brut obtenu de l'oxydation est extrait du mélange de produits bruts avec un solvant et ensuite, expulsé du solvant avec de l'hydrogène.

16. Procédé selon l'une quelconque des revendications 1 à 15, **caractérisé en ce que** l'agent d'absorption est choisi parmi le groupe consistant en le phosphate de tricrésyle, le maléate de dibutyle, des cires de poids moléculaire élevé, des hydroxcarbures aromatiques avec un poids moléculaire allant de 150 à 400 et un point d'ébullition supérieur à 140°C, de préférence le dibenzylbenzène, des diesters d'alkyle en C₁-C₄ d'acides dicarboxyliques aromatiques et aliphatiques, de préférence le 2,3-naphtalènedicarboxylate de diméthyle et/ou le 1,4-cyclohexanedicarboxylate de diméthyle, des esters méthyliques d'acides gras à longue chaine avec 14 à 30 atomes de carbone, des éthers de point d'ébullition élevé, de préférence le diméthyléther du polyéthylèneglycol, de préférence du tétraéthylèneglycol et des phtalates de dialkyle avec des radicaux alkyle en C₁-C₈, de préférence du groupe consistant en les phtalates de dialkyle avec des radicaux alkyle en C₁-C₈, en particulier le phtalate de diméthyle, le phtalate de diéthyle, le phtalate de di-n-propyle, le phtalate de diisobutyle et le phtalate de dibutyle.

17. Procédé selon l'une quelconque des revendications 1 à 16, **caractérisé en ce que** l'anhydride maléique est expulsé de l'agent d'absorption sous vide ou à des pressions qui se situent à la pression d'hydrogénation ou au maximum à 10% au-dessus de cette pression.
